# EUROPÄISCHE PATENTSCHRIFT

(11) **EP 1 322 248 B1**
(45) Veröffentlichungstag und Bekanntmachung des Hinweises auf die Patenterteilung: **07.04.2004**
(21) Anmeldenummer: 01969076.7
(22) Anmeldetag: 02.10.2001
(51) Int. Cl.: A61C 1/00

(54) **LASERVORRICHTUNG**
LASER DEVICE
DISPOSITIF LASER

(30) Priorität: 02.10.2000 AT 16672000
(43) Veröffentlichungstag der Anmeldung: 02.07.2003
(73) Patentinhaber: Femtolasers Produktions GmbH, 2100 Korneuburg (AT)
(72) Erfinder: STINGL, Andreas, A-2100 Korneuburg (AT); KRAUSZ, Ferenc, A-2331 Vösendorf (AT)
(74) Vertreter: Weinzinger, Arnulf, Dipl.-Ing.
(86) Internationale Anmeldenummer: PCT/AT2001/000309
(87) Internationale Veröffentlichungsnummer: WO 2002/028305

(56) Entgegenhaltungen:
- EP-A- 0 514 898
- EP-A- 0 654 251
- WO-A-92/04871
- DE-U- 9 208 617
- RUTI L ET AL: "DIODE-PUMPED ND:YLF ALL-IN-ONE LASER" OPTICS LETTERS, OPTICAL SOCIETY OF AMERICA, WASHINGTON, US, Bd. 20, Nr. 14, 15. Juli 1995 (1995-07-15), Seiten 1541-1543, XP000514995 ISSN: 0146-9592 in der Anmeldung erwähnt

## Beschreibung

Die Erfindung betrifft eine zwischen verschiedenen Betriebsarten umschaltbare Dentallaseranordnung.

Eine solche Dentallaseranordnung ist bereits aus der WO 98/41355 A bekannt (eine ähnliche Laseranordnung ist in der WO 95/32679 A beschrieben); dabei wird die Laserstrahlung für die Abtragung von hartem Zahngewebe eingesetzt, und es ist überdies vorgesehen, für verschiedene Anwendungen (z.B. oberflächliche Abtragung und tiefe Abtragung) verschiedene Laserstrahlungen, mit verschiedenen Wellenlängen, vorzusehen, und demgemäß sollen verschiedene Laserquellen eingesetzt werden, zwischen denen im Betrieb umgeschalten wird. Diese Verwendung von mehreren Laserquellen sowie die weiters - gemäß WO 98/41355 A - vorgesehene Verwendung von eigenen optischen Fasern für jede Laserstrahlung ist jedoch ziemlich aufwendig.

An sich sind verschiedene Einsatzmöglichkeiten für Laser in der Dentalmedizin gegeben. Beispielsweise ist zusätzlich zur Abtragung von Zahngewebe eine Versiegelung von Oberflächen von hartem Gewebe mit kurzen Laserimpulsen denkbar, wobei ebenso wie beim Abtragen eine Überhitzung des Zahngewebes zu vermeiden ist. Andererseits sind thermische Wechselwirkungen zwischen Laserstrahl und Gewebe denkbar und erwünscht, nämlich dann, wenn eine Eiweiß-Koagulation zur Stoppung von Blutungen gewünscht wird.

Wie erwähnt wurden in der Vergangenheit für unterschiedliche Behandlungen in der Dentalmedizin verschiedene Laserquellen, eingesetzt, wobei auch das Wechseln zwischen diesen Systemen aufwendig ist, insbesondere wenn zusätzlich Teile, wie Handstücke, getauscht werden müssen.

Es sind andererseits bereits Kurzpuls-Laservorrichtungen bekannt, mit denen in einem modenverkoppelten Zustand kurze Laserimpulse mit hoher Energie erzeugt werden. Solche Kurzpuls-Laservorrichtungen werden beispielsweise für Materialbearbeitungen oder aber für wissenschaftliche Arbeiten eingesetzt, und es wurde auch bereits eine Verwendung in der Dentalmedizin vorgeschlagen, vgl. US 5 742 634 A. Hinsichtlich der Erzeugung von kurzen Laserimpulsen kann beispielsweise auch noch auf die WO 98/10494 A sowie auf A. Stingl et al.: Generation of 11-fs pulses from a Ti:sapphire laser without the use of prisms; Optics Letters Vol. 19, Nr. 3, 1. Februar 1994, S. 204-206, verwiesen werden.

Die Standardtechnik zur Erzeugung von kurzen Laserimpulsen mit hoher Energie beruht auf der Technik eines Laseroszillators und eines Laserverstärkers. Der Laseroszillator, der eine Pumpeinheit enthält, der beispielsweise ein Laser-Pumpstrahl zugeführt wird, oder in der ein Diodenlaser vorgesehen ist, erzeugt eine Folge von kurzen Laserimpulsen mit niedriger Energie, beispielsweise mit einer Wiederholungsfrequenz im Bereich von einigen zehn MHz. Aus diesen Oszillator-Laserimpulsen werden Impulse mit einer niedrigeren Wiederholungsfrequenz selektiert und in einem regenerativen oder sog. Multipass-Verstärker zu Impulsen mit hoher Energie verstärkt.

Bekannt ist auch das sog. "All-in-one"-Laserkonzept, bei dem nur eine Laservorrichtung vorgesehen ist, welche sowohl als Oszillator als auch als regenerativer Verstärker eingesetzt wird, indem einfach die Pulsformung (bei niedrigen Energien) einerseits und die Verstärkung (auf hohe Energien) andererseits zu verschiedenen Zeiten vorgenommen werden. Bekannte Laservorrichtungen dieser Art (vgl. z.B. L. Turi, T. Juhasz: Diode-pumped Nd:YLF all-in-one laser; Optics Letters Vol. 20, Nr. 14, 15. Juli 1995, S. 1541-1543), mit einer Oszillatorfunktion ebenso wie einer Verstärkerfunktion, setzen aktive Modenverkopplung mit einem akusto-optischen Modulator ein.

Ein demgegenüber modifizierter All-in-one-Laser ist ferner in der älteren, nicht vorveröffentlichten Anmeldung WO 01/05002 A (PCT/AT00/00173) beschrieben.

Es ist nun Aufgabe der Erfindung, eine Dentallaseranordnung wie eingangs angeführt vorzusehen, mit der in vorteilhafter Weise ein Umschalten zwischen den verschiedenen Betriebsarten oder Behandlungsarten ausgehend von einem einzigen Lasersystem möglich ist, so dass beispielsweise bei einer Zahnbehandlung ein unmittelbares Umschalten von einem Betrieb, in dem Zahngewebe abgetragen wird, auf einen Betrieb möglich ist, in dem eine Blutung durch Eiweißkoagulation gestoppt wird, bzw. gegebenenfalls auch auf einen Betrieb, in dem eine Versiegelung von offenen Stellen an einem Zahn möglich ist. Dieser Wechsel soll dabei insbesondere durch einfaches Umschalten, beispielsweise mit einem mechanischen Schalter, ermöglicht werden.

Die erfindungsgemäße umschaltbare Dentallaseranordnung ist demgemäß dadurch gekennzeichnet, dass eine als Laser-Oszillator und als Laser-Verstärker betreibbare Laservorrichtung (All-inone-Laservorrichtung) mit einem Laserresonator, der eine Pumpeinheit und einen aktiven Modenkoppler sowie ein selektive Verluste im Resonator einführendes optisches Verlustelement aufweist, und mit einer Laserstrahl-Auskopplungseinheit, wobei der Modenkoppler an eine Steuerschaltung angeschaltet ist zum Umschalten zwischen einer zum Koagulieren von weichem Gewebe vorgesehenen CW-Betriebsart, in der der Modenkoppler abgeschaltet ist, und zwei Kurzpuls-Betriebsarten, in denen von der Steuerschaltung HF-Signale mit gleicher Frequenz, jedoch unterschiedlicher Leistung an den Modenkoppler gelegt sind, um Laserimpulse vorbestimmter Dauer zu erzeugen, wobei eine der zwei Kurzpuls-Betriebsarten zum Abtragen von hartem Zahngewebe vorgesehen ist, wogegen die andere Kurzpuls-Betriebsart, in der die Laserstrahlung im Vergleich zur erstgenannten Kurzpuls-Betriebsart eine geringere Spitzenleistung aufweist, zum Oberflächenversiegeln von hartem Zahngewebe vorgesehen ist, und wobei ein Ausgang der. Steuerschaltung weiters an das ansteuerbare optische Verlustelement zur Steuerung der Verluste im Resonator in den Kurzpuls-Betriebsarten gelegt ist.

Die vorliegende Dentallaseranordnung basiert somit auf dem vorstehend beschriebenen All-in-one-Konzept, d.h. es wird eine einzige Laservorrichtung eingesetzt, die mit Hilfe der Steuerschaltung zwischen den verschiedenen Betriebsarten so umgeschaltet werden kann, dass sie entweder als CW-(CW - Continuous Wave - Dauerstrich)-Lasersystem oder als Kurzpuls-Lasersystem arbeitet, wobei hier wiederum verschiedene Betriebsarten vorgesehen sind, die ebenfalls mit Hilfe der Steuerschaltung eingestellt werden können. In der CW-Betriebsart wird ein CW-Laserstrahl erzeugt, wobei der Modenkoppler abgeschaltet ist, d.h. es wird kein Steuersignal von der Steuerschaltung an den Modenkoppler angelegt. Der CW-Laserstrahl wird hinsichtlich seiner Ausgangsleistung beispielsweise durch entsprechende Ansteuerung der Auskopplung festgelegt. Andererseits wird in den Kurzpuls-Betriebsarten ein entsprechendes Hochfrequenzsignal mit der erforderlichen Leistung an den Modenkoppler gelegt, um die gewünschten kurzen Impulse zu erzeugen. Je nach Leistung des jeweils an den Modenkoppler angelegten HF-Signals bilden sich Laserimpulse mit längerer oder kürzerer Dauer aus. In diesen Kurzpuls-Betriebsarten wird ferner die Laserverstärker-Funktion bewirkt, um wie einleitend beschrieben einzelne Kurzpulse nach Aufschaukeln auf hohe Energien nutzen zu können. Dabei werden vorübergehend Verluste in das Lasersystem eingeführt, die die Pumpeinheit durch laufende Anhebung der Verstärkung auszugleichen sucht. Wenn dann die Verluste bei einem hohen Verstärkungsgrad plötzlich weggeschaltet werden, wird der Laser-Kurzpuls innerhalb weniger Durchläufe im Resonator zu Folge der noch hohen Verstärkung in der Pumpeinheit auf eine hohe Energie gebracht und sodann zum Zeitpunkt der Sättigung ausgekoppelt. Von besonderem Vorteil ist hier auch, dass das optische Verlustelement mehrfach ausgenützt werden kann, nämlich einerseits zur Leistungssteuerung im Dauerstrichbetrieb und andererseits als Steuer- und Schaltelement in den Kurzpuls-Betriebsarten. Das ansteuerbare optische Verlustelement kann dabei einfach durch einen den Grad der Auskopplung des Laserstrahls steuernden optischen Schalter, insbesondere durch eine Pockelszelle in Verbindung mit einem λ/4-Plättchen, gebildet sein. Dieser optische Schalter bzw. diese Pockelszelle führt in einem längeren Rhythmus dadurch Verluste in die Laserstrahlung ein, dass ein Teil der Laserstrahlung (durch entsprechende Drehung ihrer Polarisationsebene) an einem Strahlteiler ausgekoppelt wird; bei Anlegen eines λ/4-Spannungssignals an die Pockelszelle in Verbindung mit dem λ/4-Plättchen bzw. bei Abschalten der Spannung am optischen Schalter wird zufolge der sich jeweils ergebenden Ausrichtung der Polarisationsebene des Laserstrahls ein Hintanhalten der Auskopplung bzw. ein Auskoppeln des Kurzpulses zum gewünschten Zeitpunkt erreicht.

Um demgemäß den Grad der Auskopplung des Laserstrahls zwecks Einführung von Verlusten bzw. Verstärkung der erzeugten Kurzpulse und deren Auskopplung auf zweckmäßige Weise zu erzeugen, ist es von Vorteil, wenn die Steuerschaltung zur Abgabe unterschiedlich hoher Spannungen an die Pockelszelle, je nach eingeschalteter Betriebsart, schaltbar ist. Für das kurzfristige Aufschaukeln der Laserpulse in der Verstärkungsphase (ohne Auskoppeln von Laserstrahlung) sowie das bei Sättigung erfolgende Auskoppeln der energiereichen Kurzpulse ist es weiters günstig, wenn die Steuerschaltung zur Abgabe gesonderter Spannungs-EIN- und AUS-Schaltsignale an die Pockelszelle eingerichtet ist. Um die verschiedenen Schaltvorgänge dabei phasenrichtig vorzunehmen, ist es überdies vorteilhaft, wenn die EIN- und AUS-Schaltsignale in der Steuerschaltung aus dem ihr zugeführten HF-Signal durch Frequenzteilung und mit Hilfe unterschiedlicher Verzögerungsglieder hergeleitet werden.

Der Modenkoppler kann beispielsweise ein akusto-optischer Modenkoppler sein, und er bewirkt die Bildung von Laserimpulsen, deren Frequenz grundsätzlich durch die Frequenz des HF-Signals bestimmt ist, wobei die Frequenz natürlich auf die Länge des Resonators abgestimmt sein muss und beispielsweise bei gängigen Resonator-Längen (und damit Laufzeiten der Laserimpulse im Resonator) 75 oder 80 MHz betragen kann. Hingegen treten in den Kurzpuls-Betriebsarten die verstärkten Laserpulse, die mit Energie angereichert wurden, mit einer Wiederholungsfrequenz von beispielsweise 5 kHz auf.

Um in den verschiedenen Kurzpuls-Betriebsarten Laserstrahlung mit unterschiedlicher Energie zur Verfügung zu haben, ist der Modenkoppler von der Steuerschaltung durch HF-Signale mit gleicher Frequenz, jedoch unterschiedlicher Leistung schaltbar. Je nach Leistung des an den Modenkoppler angelegten HF-Signals bilden sich Kurzpulse mit entsprechender kürzerer oder längerer Pulsdauer aus, und diese Kurzpulse werden in der Folge auf die beschriebene Weise auf höhere oder weniger hohe Spitzenleistungen verstärkt. Dies geschieht dabei ohne weitere Einstellung der Laservorrichtung von selbst. Die Durchschnitts-Ausgangsleistung bleibt dabei naturgemäß gleich. Für die Steuerung der Leistung des HF-Signals ist es dabei günstig, wenn die Steuerschaltung einen HF-Generator mit einem regelbaren Leistungsverstärker enthält.

Um das Umschalten zwischen den einzelnen Betriebsarten möglichst einfach zu gestalten, kann die Steuerschaltung an einen Betriebsarten-Wählschalter, z.B. einen Fußschalter; angeschlossen sein. Je nach Stellung des Wählschalters gibt die Steuerschaltung die entsprechenden Steuersignale an die verschiedenen Komponenten der Laservorrichtung, wie insbesondere an den Modenkoppler bzw. an den optischen Schalter, ab, um die gewünschte Betriebsart einzustellen. Auf diese Weise kann wie erwähnt die genannte CW-Laserstrahlung für ein "Koagulieren" von weichem Gewebe bzw. eine Kurzpuls-Erzeugung bewirkt werden, wenn in einer Betriebsart "Abtragen" ("Ablation") ein HF-Signal entsprechender Leistung an den Modenkoppler gelegt wird, um die gewünschten Kurzpulse zu erzeugen. In einer Betriebsart "Oberflächenversiegeln" wird die Leistung des an den Modenkoppler angelegten HF-Signals reduziert, so dass sich Laserimpulse mit größerer Dauer ausbilden, wobei die Laserstrahlung eine geringere Spitzenleistung hat.

Eine Koagulationswirkung wird am besten mit einem CW-Laserstrahl mit einer Leistung bis zu 3 W erhalten, wobei die Position des Laser-Fokus fest bleiben kann. Zum Abtragen von Zahngeweben wird, wie Untersuchungen gezeigt haben, bevorzugt eine Pulsdauer von weniger als 1 ps verwendet. Hingegen werden zur Oberflächenversiegelung Pulsdauern von ungefähr 100 ps vorgesehen. Eine solche Oberflächenversiegelung wird vom Zahnarzt vorgenommen, um offene Stellen des Zahns zu versiegeln. Zum Abtragen von Zahngewebe ebenso wie bei der Oberflächenversiegelung ist eine Erwärmung des Zahns zu vermeiden, wobei bei der Behandlung von Karies Temperaturanstiege von mehr als 5°C nicht nur Schmerzen, sondern auch irreversible Schädigungen der Pulpa verursachen können. Zu diesem Zweck kann der Laserstrahl bzw. sein Fokus am Zahn hin und her bewegt werden.

Für die Steuerung des Laserstrahls auf bestimmte Zielpunkte hin ist es ferner vorteilhaft, wenn im Weg des ausgekoppelten Laserstrahls ein die Frequenz eines Teils des Laserstrahls verdoppelnder Frequenzverdoppler zum Vorsehen eines sichtbaren Lichtstrahls vorgesehen ist. Weiters ist es für eine selektive Abschwächung der Laserstrahl-Leistung günstig, wenn im Weg des ausgekoppelten Laserstrahls ein steuerbarer Abschwächer, z.B. eine Scheibe mit Teilen unterschiedlicher Reflektivität oder ein aus Filtermaterial gebildeter Keil, angeordnet ist.

Die Erfindung wird nachstehend anhand von bevorzugten Ausführungsbeispielen, auf die sie jedoch nicht beschränkt sein soll, und unter Bezugnahme auf die Zeichnung noch weiter erläutert. In der Zeichnung zeigen im Einzelnen:
Fig. 1 schematisch eine Dentallaseranordnung, wobei nur ganz schematisch ein Handstück sowie ein zu behandelnder Zahn veranschaulicht sind;
Fig. 2 ein Schema der dabei eingesetzten All-in-one-Laservorrichtung mit ihren einzelnen optischen Komponenten;
Fig. 3 ein Schema dieser Laservorrichtung mit den einzelnen Komponenten in der Art eines Blockschaltbildes;
Fig. 4 mit den Fig. 4a und 4b ein Diagramm mit einzelnen HF-Signal-Leistungspegeln (Fig. 4a) und Steuerspannungen (Fig. 4b) für die verschiedenen Betriebsarten der Laservorrichtung;
Fig. 5 ein gegenüber Fig. 3 detaillierteres Schaltbild von Teilen der Steuerkomponenten der Laservorrichtung;
Fig. 6 in Teil-Diagrammen Fig. 6a bis 6f die Herleitung von verschiedenen Signalen mit Hilfe der Komponenten von Fig. 5;
Fig. 7 ein Diagramm zur Veranschaulichung des Vorganges beim Verstärken der Laserimpulse;
Fig. 8 ein Schema zur Veranschaulichung der Arbeitsbereiche für die verschiedenen Betriebsarten der Laservorrichtung in einem Diagramm "Spitzenleistung" über "Einwirkzeit"; und
die Fig. 9 und 10 schematisch je die Anordnung eines Frequenzverdopplers und eines steuerbaren Abschwächers im Weg des ausgekoppelten Laserstrahls, um zusätzlich einen Pilot-Laserstrahl mit sichtbarem Licht für die Sichtbarmachung des Zielpunktes sowie die Möglichkeit einer Laserstrahl-Leistungsabschwächung vorzusehen.

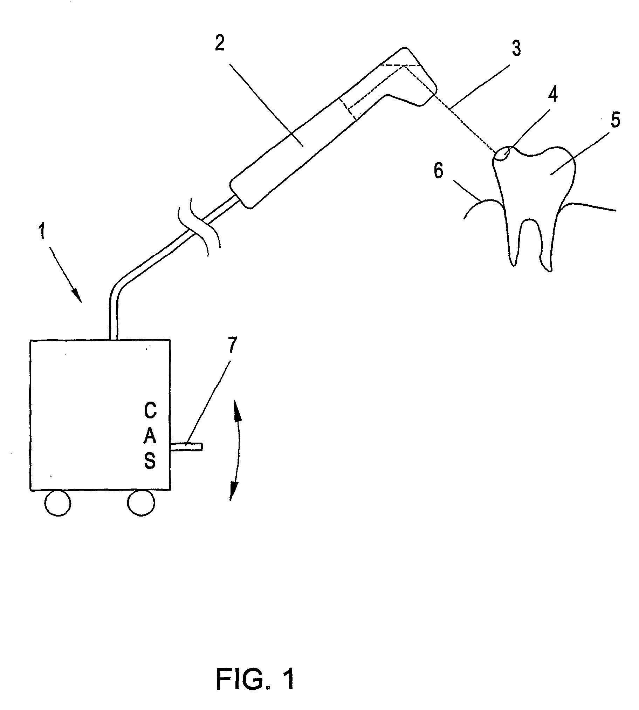

In Fig. 1 ist nur ganz schematisch und nicht maßstäblich eine dentalmedizinische Laseranordnung mit einer Laservorrichtung 1 veranschaulicht, an die in an sich herkömmlicher Weise über Gelenkarme und dergl. (nicht gezeigt) ein Handstück 2 angeschlossen ist, mit dem ein therapeutischer Laserstrahl 3 auf die zu behandelnde Stelle 4 an einem Zahn 5 gerichtet wird. Die Behandlungsstelle 4 kann auch im Bereich des Zahnfleisches 6 liegen, wenn dort eine Blutung durch Koagulieren von Eiweiß gestoppt werden soll.

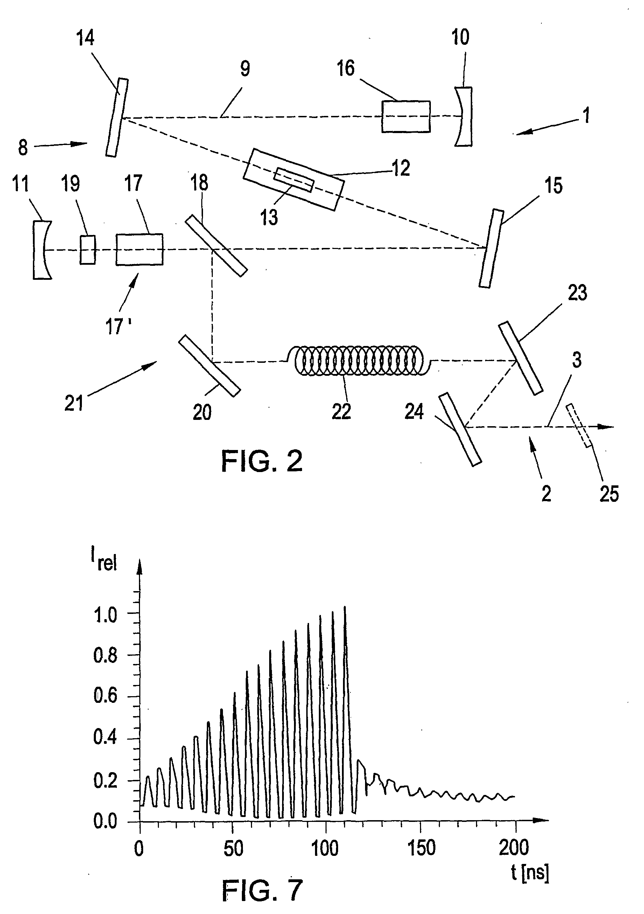

Der Laserstrahl 3 wird von der Laservorrichtung 1 dem Handstück 2 über in Fig. 1 in nicht näher dargestellte Lichtleiter (vgl. auch Pos. 22 in Fig. 2) zugeführt, wie dies an sich bekannt ist.

Die Laservorrichtung 1 weist einen Fußschalter 7 auf, der beispielsweise drei Stellungen, entsprechend drei Betriebsarten der Laservorrichtung 1, hat, nämlich für (1) Koagulieren (Betriebsart C); (2) Abtragen (Betriebsart A); und (3) Oberflächenversiegeln (Betriebsart S).

In Fig. 2 ist die Laservorrichtung 1 hinsichtlich ihrer optischen Komponenten näher veranschaulicht. Die Laservorrichtung 1 ist dabei auf Basis eines All-in-one-Lasers aufgebaut, d.h. sie fungiert als Oszillator und als Verstärker, wobei ein einzelner Resonator 8 für die Erzeugung des Laserstrahls 9 im Resonator 8 in der jeweiligen. Betriebsart vorgesehen ist.

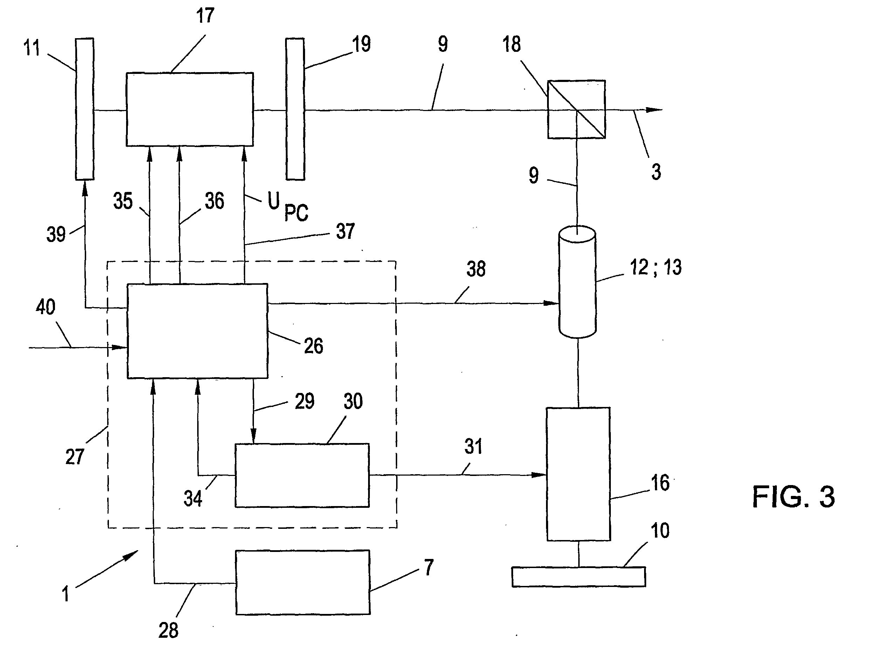

Im Einzelnen sind zwei Resonator-Endspiegel 10, 11 vorgesehen, zwischen denen sich der Laserstrahl 9 im Resonator 8 der Laservorrichtung 1 aufbaut. Im Resonator 8 ist eine Pumpeinheit 12 mit einem z.B. Dioden-gepumpten Laserkristall 13 enthalten, welche nur schematisch angedeutet und an sich bekannt ist; diese Pumpeinheit 12 ist zwischen zwei Laserspiegeln 14, 15 (in der Art einer Z-Faltung) angeordnet. Benachbart dem einen Endspiegel 10 ist weiters ein aktiver Modenkoppler 16, beispielsweise ein akusto-optischer Modulator, angeordnet, der eine aktive Modenverkopplung bewirkt, wenn eine entsprechende Ansteuerung erfolgt, wie dies nachstehend noch näher anhand der Fig. 3 bis 6 erläutert werden wird.

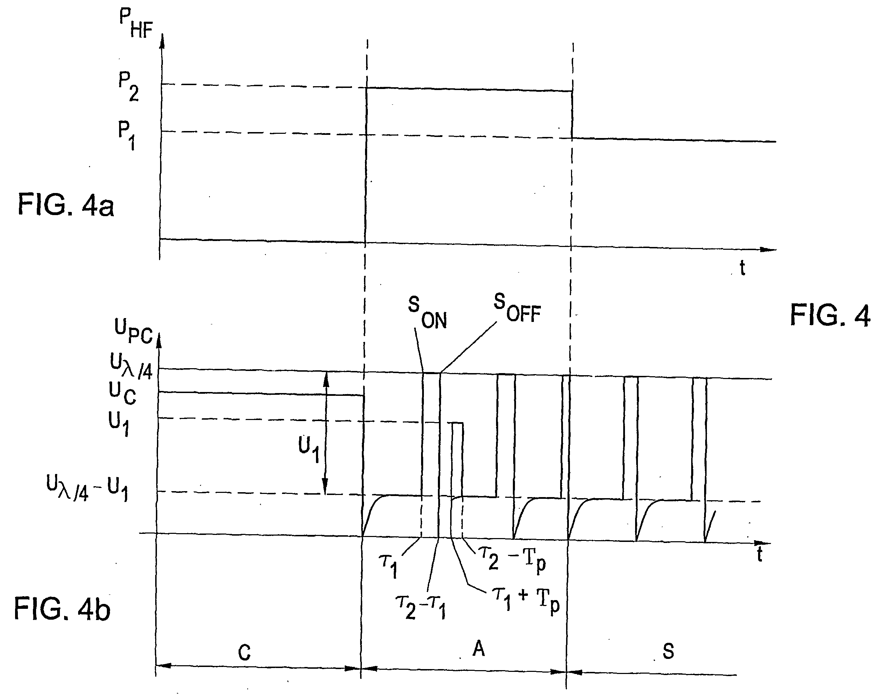

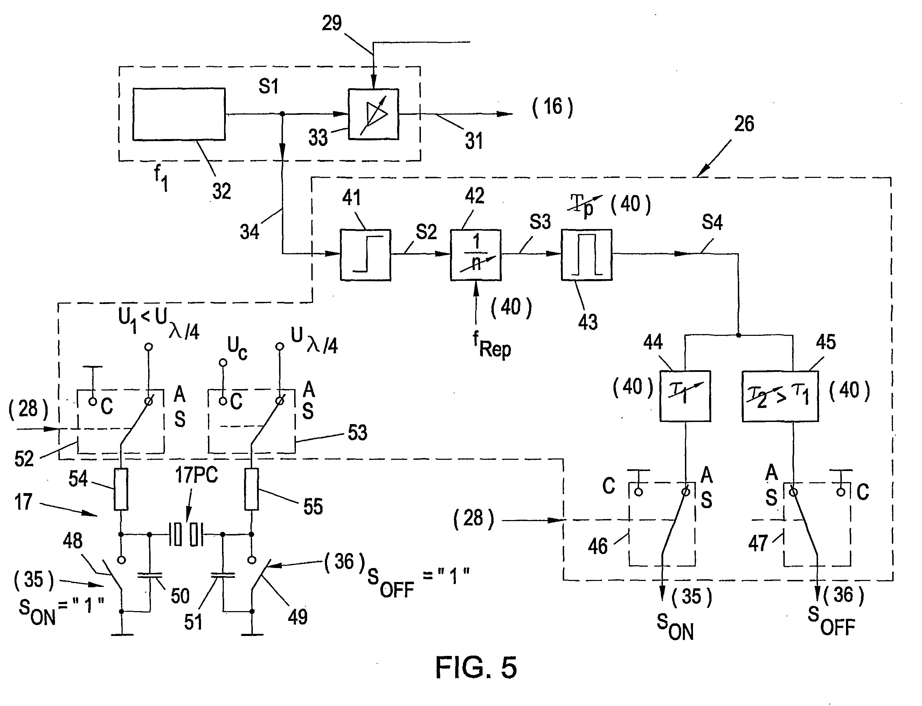

Im Weg des Laserstrahls 9 ist weiters benachbart dem anderen Endspiegel 11 des Resonators 8 ein optischer Schalter, hier in Form einer Pockelszelle 17, vorgesehen, wobei mit diesem optischen Schalter bzw. mit dieser Pockelszelle 17 in den Kurzpuls-Betriebsarten A, S Verluste in die Laserstrahlung eingeführt werden. Diese Funktion als optisches Verlustelement 17' wird mit der Pockelszelle 17 bzw. allgemein dem optischen Schalter dadurch erreicht, dass der Laserstrahl 9 in unterschiedlichem Ausmaß ausgekoppelt wird, s. den Laserstrahl 3, wobei der Grad der Auskopplung hier über die Drehung der Polarisationsebene des Laserstrahls 9 und demzufolge die Anteile des Laserstrahls 9, die an einem polarisationsabhängigen Strahlteiler 18 durchgelassen bzw. reflektiert werden, gesteuert wird. Die Pockelszelle 17 wird demgemäß in an sich bekannter Weise selektiv als Polarisationsdreher eingesetzt, und zum Auskoppeln der Laserstrahlung über den Strahlteiler 18 bei entsprechender Drehung der Polarisationsebene des Laserstrahls 9 ist weiters im Strahlengang des Laserstrahls 9 ein λ/4-Plättchen 19 vorgesehen, das eine entsprechende Drehung der Polarisationsebene des Laserstrahls 9 (insgesamt 90° am λ/4-Plättchen 19 bei einem Hin- und Rücklauf des Laserstrahls 9) bewirkt. Zwecks - teilweiser - Auskopplung des Laserstrahls 9 am Strahlteiler 18 zu einem Spiegel 20 hin wird die Pockelszelle 17 entsprechend angesteuert, um durch die dann an ihr bewirkte Drehung der Polarisationsebene des Laserstrahls 9 (zusätzlich zur Drehung durch das λ/4-Plättchen 19) den Laserstrahl 9 am Strahlteiler 18 mehr oder weniger stark zur Reflexion zu bringen, wie nachstehend anhand der Fig. 4 und 5 noch näher erläutert wird. Auf diese Weise ist eine in Fig. 2 allgemein mit 21 bezeichnete Auskopplungseinheit realisiert.

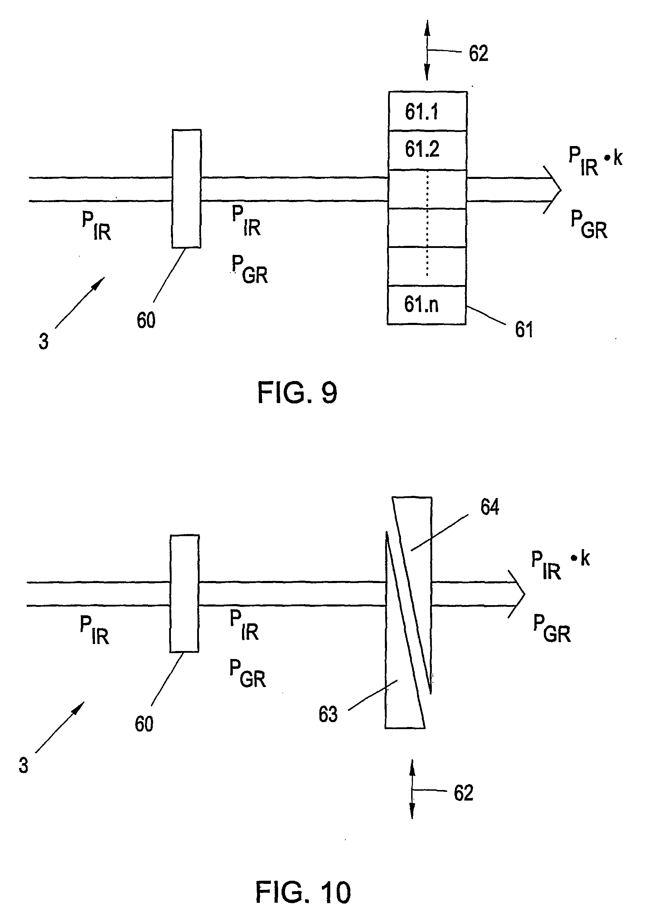

Der ausgekoppelte Laserstrahl 3 wird dem Handstück 2 über einen Lichtleiter 22 sowie über zwei Gitter 23, 24 zugeführt, welche ein Komprimieren der Laserpulse bewirken. Im Strahlengang nach diesen beiden Gittern 23, 24 können noch optische Elemente 25 zum Vorsehen eines sichtbaren Pilotstrahls und/oder einer steuerbaren Abschwächung vorgesehen sein, wie dies nachfolgend anhand der Fig. 9 und 10 noch weiter erläutert wird.

In Fig. 3 sind die vorstehend erwähnten Laserkomponenten in einem gegenüber Fig. 2 etwas vereinfachten Schema gezeigt, und zusätzlich sind die zugehörigen elektronischen Komponenten in der Art eines Blockschaltbildes veranschaulicht. Dabei wurden auch die Positionen des λ/4-Plättchens 19 und der Pockelszelle 17 vertauscht, um zu zeigen, dass die relative Position dieser zwei Komponenten 19, 27 nicht weiter von Bedeutung ist, um die gewünschte Auskopplung des Laserstrahls zum vorgegebenen Zeitpunkt zu erzielen.

Die verschiedenen Komponenten, wie insbesondere der Modenkoppler 16 und die Pockelszelle 17, werden elektronisch von einer Steuereinheit 26 einer elektrischen Steuerschaltung 27 angesteuert, die mit dem Fußschalter 7 für die Wahl der jeweiligen Betriebsart (Koagulieren; Abtragen; Versiegeln) über eine Eingangsoder Steuerleitung 28 verbunden ist. Die Steuereinheit 26 gibt dann ein entsprechendes Steuersignal über einen Ausgang 29 an einen HF-Generator 30 ab, welcher entweder kein Signal oder eines von zwei HF-Signalen, mit unterschiedlichen Leistungspegeln P₁ bzw. P₂ (s. Fig. 4a), über eine Leitung 31 an den Modenkoppler 16 abgibt. Diese beiden Leistungspegel P₁, P₂ sind im Diagramm von Fig. 4a schematisch gezeigt, wobei das HF-Signal mit der höheren Leistung P_{HF}=P₂ in der Betriebsart "Abtragen" abgegeben wird, wogegen das HF-Signal mit der etwas niedrigeren Leistung P₁ in der Betriebsart "Oberflächenversiegeln" abgegeben wird. In der CW-Betriebsart "Koagulieren" wird kein HF-Signal an den Modenkoppler 16 angelegt, d.h. P_{HF}=0. Im unteren Teil von Fig. 4 sind die Betriebsarten "Koagulieren" bei C, "Abtragen" bei A und "Oberflächenversiegeln" bei S veranschaulicht. Der HF-Generator 30 kann dazu eine entsprechende Umschaltmöglichkeit bzw. Ansteuerungsmöglichkeit im Bereich seiner Endstufe (in Fig. 3 nicht gezeigt), wie an sich herkömmlich, aufweisen, und dies ist in Fig. 5 veranschaulicht, wobei der eigentlichen HF-Generatorschaltung 32 ein regelbarer Leistungsverstärker 33 nachgeschaltet ist, der je nach dem Steuersignal am Eingang (Leitung 29) ein HF-Signal mit der Leistung P₂ oder mit der Leistung P₁ oder kein HF-Signal an den Modenkoppler (Modelocker) 16 abgibt.

Das HF-Signal, das vom HF-Generator 30 abgegeben wird, hat in den beiden Kurzpuls-Betriebsarten A, S die selbe Frequenz, z.B. 75 MHz oder 80 MHz, je nach der Länge des Resonators 8, d.h. je nach der Weglänge der Laserpulse zwischen den Endspiegeln 10, 11.

Gemäß Fig. 3 und 5 erhält die Steuereinheit 26 weiters vom HF-Generator 30 ein Signal betreffend Phaseninformation auf einer Leitung 34 zugeführt, wie nachfolgend noch näher beschrieben wird.

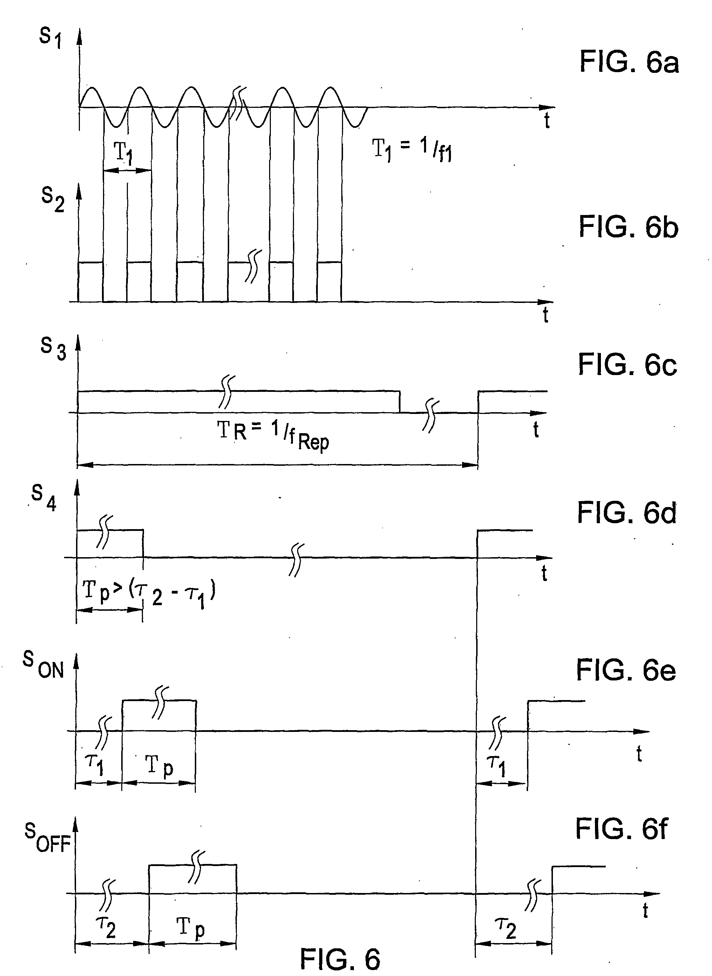

Die Steuereinheit 26 steuert weiters über "Aus"- bzw. "Ein"-Steuerleitungen 35 bzw. 36 (s. Fig. 3) die Pockelszelle 17 an, und über eine Leitung 37 wird der Pockelszelle 17 die erforderliche Hochspannung U_{PC}, z.B. die sog. λ/4-Spannung U_{λ/4} für eine Drehung der Polarisationsebene des Laserstrahls 9 um 90° bei jedem Hin- und Rücklauf, angelegt, vgl. auch Fig. 4b, um die gewünschten verstärkten Kurzpulse in der All-in-one-Laservorrichtung 1 während des Betriebs als Verstärker zu erzeugen, vgl. auch die nachfolgenden Ausführungen anhand der Fig. 5 und 6.

Die Steuereinheit 26 sorgt ferner über eine Leitung 38 zur Pumpeinheit 12 mit der Diode 13 noch dafür, dass die Diode 13 den benötigten Diodenstrom erhält, wie dies an sich bekannt ist. Weiters kann die Steuereinheit 26 dazu eingesetzt werden, den einen Resonator-Endspiegel 11 über eine Steuerleitung 39 zu justieren, um so die Resonatorlänge exakt einzustellen.

Über einen Eingang 40 können der Steuereinheit 26 Zeitsteuerparameter sowie Vorgaben hinsichtlich der Repetitionsrate (Pulswiederholungsfrequenz fᵣₑₚ) zugeführt werden.

Im Betrieb wird in der Betriebsart C "Koagulieren" der Modenkoppler 16 abgeschaltet, d.h. es wird ihm kein HF-Signal vom HF-Generator 30 zugeführt. Das Lasersystem arbeitet im Niederleistungsbereich, wobei ein CW-Laserstrahl 9 erzeugt wird, welcher einen Anteil hat, der entsprechend dem polarisierenden Strahlteiler 18 in der zeichenebene von Fig. 2 linear polarisiert ist. An die Pockelszelle 17 wird dabei eine Spannung U_{PC} = U_{c} (s. Fig. 4) angelegt, die zwischen 0 V und der λ/4-Spannung U_{λ/4} liegt. Hierbei fungiert der polarisierende Strahlteiler 18 als teilweise reflektierender Auskoppler, wobei je nach der Größe der Spannung U_{c} und damit je nach Größe der Drehung der Polarisationsebene des Laserstrahls 9 durch die Pockelszelle 17 in Verbindung mit der Polarisationsdrehung durch das λ/4-Plättchen 19 z.B. 20% der Laserstrahlung ausgekoppelt werden. Der Laserstrahl 9 wird im Einzelnen vom Strahlteiler 18 durchgelassen, wenn er von der Pumpeinheit 12 kommt, und er wird auf dem Weg vom Endspiegel 11 zurück teilweise vom Strahlteiler 18 reflektiert. Die CW-Leistung wird, wie sich aus Vorstehendem ergibt, durch entsprechende Wahl der an die Pockelszelle 17 angelegten Spannung U_{PC} = U_{c} - entsprechend dem Grad der Auskopplung - eingestellt. Wie bereits erwähnt beträgt in dieser Betriebsart C "Koagulieren" die Leistung zweckmäßigerweise bis zu 3 W. Der ausgekoppelte Laserstrahl 3 wird in dieser Betriebsart C in einem festen Fokus an der Behandlungsstelle (4 in Fig. 1) fokussiert.

In den Kurzpuls-Betriebsarten A und S (Abtragen, Versiegeln) wird der Modenkoppler 16 der Laservorrichtung 1 aktiviert, wobei wie dargelegt ein HF-Signal mit einer bestimmten Leistung, je nach Betriebsart (A bzw. S), von der Steuerschaltung 27 bzw. genauer von deren HF-Generator 30 an den Modenkoppler 16 angelegt wird, um Kurzpulse mit einer Dauer von beispielsweise ungefähr 1 ps (Betriebsart A "Abtragen") bzw. ungefähr 100 ps (Betriebsart S "Versiegeln"), in Entsprechung zur angelegten HF-Leistung, P₂ bzw. P₁ zu erzeugen.

Aus der Reihe von Kurzpulsen, die mit der Frequenz des HF-Signals erzeugt werden, werden in der Verstärkungsphase der Laservorrichtung 1 einzelne Pulse selektiert und beim mehrmaligen Durchlaufen des Resonators 8 verstärkt, wie dies an sich bekannt ist.

Im Einzelnen werden dabei in dieser Betriebsart der Laservorrichtung 1 in einer ersten Phase Verluste dadurch eingeführt, dass ein größerer Anteil der Laserstrahlung, z.B. 50-60%, aus dem Resonator 8 durch Anlegen einer entsprechenden Spannung an die Pockelszelle 17 ausgekoppelt werden. Dadurch wird, um diese Verluste zu kompensieren, eine hohe Verstärkung im Laserkristall 13 der Pumpeinheit 12 erzwungen. Die ausgekoppelte Laserstrahlung ist am Handstück 2 bloß als ein unwesentlicher Hintergrund mit geringer Energie anzusehen.

Sobald eine Sättigung erreicht wird, wird an die Pockelszelle 17 die λ/4-Spannung U_{λ/4} angelegt, und zwar zu einem Zeitpunkt, in dem sich der Laserimpuls im Resonator 8 gemäß Fig. 2 rechts von der Pockelszelle 17 befindet, etwa wenn er im Bereich des Modenkopplers 16 vorliegt. Dadurch wird beim nachfolgenden Durchlauf des Laserpulses hin zum Endspiegel 11 und zurück zweimal eine λ/2-Drehung der Polarisationsebene insgesamt erreicht, so dass keine Auskopplung des Laserstrahls 9 mehr erfolgt. Die Verstärkung in der Pumpeinheit 12 ist zu diesem Zeitpunkt jedoch hoch, und der Laserpuls schaukelt sich in 20 bis 30 Durchläufen, was seine Energie anlangt, auf, wobei aber gleichzeitig die Verstärkung in der Pumpeinheit 12 allmählich abgebaut wird, um einen stationären Zustand zu erreichen. Bei Sättigung wird die Pockelszelle 17 ausgeschaltet, d.h. keine Hochspannung U_{PC} mehr an sie angelegt, und zufolge der Polarisationsebenen-Drehung nur im λ/4-Plättchen 19 wird jetzt eine praktisch 100%ige Auskopplung des Laserstrahls 9 am Strahlteiler 18 bewirkt; der ausgekoppelte Laserpuls 3 wird dem Handstück 2 zugeführt. Auch hier gilt wiederum, dass die Pockelszelle 17 zu dem Zeitpunkt ausgeschaltet wird, wenn der Laserpuls in der Darstellung in Fig. 2 rechts von ihr im Resonator 8 vorliegt.

Eine entsprechende Information über den Ort des Laserpulses (Phaseninformation) kann aus dem Modenkoppler-Signal hergeleitet werden, da beispielsweise im Maximum des HF-Signals, das an den Modenkoppler 16 angelegt wird, der Laserimpuls im Modenkoppler 16 vorliegt. Diese Phaseninformation betreffend die Position des Laserpulses wird wie erwähnt über die Leitung 34 vom HF-Generator 30 an die Steuereinheit 26 geliefert.

In Fig. 5 ist ein Beispiel für die Herleitung von Schaltimpulsen, die über die Leitungen 35 ("EIN") und 36 ("AUS") an die Pockelszelle 17 - zusätzlich zur jeweiligen Hochspannung U_{PC} über die Leitung 37 - von der Steuereinheit 26 abgegeben werden, näher veranschaulicht. Dabei ist ersichtlich, dass das vom HF-Generatorkreis 32 abgegebene HF-Signal S₁ mit der Frequenz f₁ noch vor dessen Verstärkung im Leistungsverstärker 33 abgezweigt und parallel zum Leistungsverstärker 33 auch als Phaseninformationssignal über die Leitung 34 der Steuereinheit 26 zugeführt wird, die in Fig. 5 mit strichlierten Linien veranschaulicht ist.

In der Steuereinheit 26 wird das HF-Signal S₁ (s. auch Fig. 6a) einer Impulsformerstufe 41 zugeführt, so dass ein Impulssignal S₂ (s. Fig. 6b) mit Rechteckimpulsen erhalten wird, das an einen einstellbaren Frequenzteiler 42 angelegt wird, der mit einem die Wiederholungsfrequenz fᵣₑₚ aufweisenden Signal (über die Leitung 40, s. auch Fig. 3) eingestellt wird. Das Signal S₃ am Ausgang dieses Frequenzteilers 42 (s. Fig. 6c) hat demgemäß eine Periode T_{R} = 1/fᵣₑₚ, im Gegensatz zur Periode T₁ = 1/f₁ des HF-Signals S₁ bzw. Impulssignals S₂. Das Impulssignal S₃ wird dann an eine monostabile Kippstufe 43 angelegt, die Impulse mit einer vorgegebenen, einstellbaren Impulsdauer T_{P} erzeugt. Dieser Impuls, d.h. das Signal S₄ (das in Fig. 6d dargestellt ist), wird sodann an zwei zueinander parallel geschaltete Verzögerungsglieder 44 bzw. 45 angelegt, wo eine jeweils einstellbare Zeitverzögerung τ₁ (Verzögerungsglied 44) bzw. τ₂ > τ₁ (Verzögerungsglied 45) eingeführt wird. Die so verzögerten Impulse S₄ werden in den Kurzpuls-Betriebsarten A bzw. S über in Fig. 5 schematisch dargestellte Schalter 46 bzw. 47 - die mit Hilfe des Fußschalters 7 über die Leitung 28 eingestellt werden - als EIN-Schaltsignal S_{ON} bzw. AUS-Schaltsignal S_{OFF} für die Pockelszelle 17 erhalten. In der Betriebsart C (Koagulieren) werden die Schalter 46, 47 in die andere Schaltposition gebracht, so dass an den Ausgängen Massepotential liegt.

Im linken Teil der Fig. 5 ist die Pockelszelle 17 mit dem Pockelszellen-Kristall 17PC gezeigt, welcher mit seinen beiden Anschlüssen an jeweils einem Schalter 48 bzw. 49 liegt: diesen Schaltern 48, 49 ist jeweils ein Kondensator 50 bzw. 51 parallel geschaltet. Der Schalter 48 wird dabei geschlossen, wenn das EIN-Schaltsignal S_{ON} (s. Fig. 6e) hoch ist ("1"); dagegen wird der Schalter 49 geschlossen, wenn das AUS-Schaltsignal S_{OFF} ("1") anliegt, d.h. hoch ist (s. Fig. 6f). Diese Schaltsignale werden wie erwähnt über die Leitungen 35 bzw. 36 in Fig. 3 zugeführt und liegen an den Schaltern 46, 47 der Steuereinheit 26 an.

Innerhalb der Steuereinheit 26 liegen weiters drei Gleichspannungen vor, nämlich die λ/4-Spannung U_{λ/4}, die demgegenüber etwas kleinere Gleichspannung U_{c} für die Betriebsart Koagulieren (Betriebsart C) und eine Spannung U₁, die ebenfalls kleiner als die λ/4-Spannung U_{λ/4} ist, vgl. außer Fig. 5 auch Fig. 4b. Weiters liegt ein Anschluss mit Masse vor, mit dem die in Fig. 5 linke Seite der Pockelszelle 17 in der Betriebsart C (Koagulieren) verbunden ist. Die rechte Seite der Pockelszelle 17 liegt in dieser Betriebsart C an der Spannung U_{C}. Zur Umschaltung auf die Kurzpuls-Betriebsarten A, S werden Schalter 52, 53 angesteuert, die in Fig. 5 nur schematisch dargestellt sind und in herkömmlicher Weise durch elektronische Schalter realisiert sein werden. Die Ansteuerung erfolgt dabei wiederum vom Fußschalter 7 her über die Steuerleitung 28, s. Fig. 3. In den Kurzpuls-Betriebsarten A, S liegt die rechte Seite der Pockelszelle 17 (gemäß Darstellung in Fig. 5) an der λ/4-Spannung U_{λ/4}, wogegen die linke Seite über den Schalter 52 an die Spannung U₁ gelegt ist. Die beiden Spannungen U₁ und U_{λ/4} werden dabei über Widerstände 54 bzw. 55 angelegt.

Wenn nun der Schalter 48 (der ebenso wie der Schalter 49 und auch wie die Schalter 46, 47 selbstverständlich durch elektronische Schalter realisiert sein kann) bei Anlegen des EINSchaltsignals S_{ON} (wenn also der Impuls S_{ON} gemäß Fig. 6e auf "1" liegt), geschlossen wird, so liegt der Stromfluss durch den Pockelszellen-Kristall 17PC vom Anschluss U_{λ/4} über den Widerstand 55, dem Pockelszellen-Kristall 17PC zu Masse vor. Wenn dann nach der Zeitdauer τ₂ - τ₁ (s. Fig. 6d, 6e und 6f) das AUS-Schaltsignal S_{OFF} auf "1" geht, wird der Schalter 49 geschlossen, so dass beide Seiten des Pockelszellen-Kristalls 17PC über die Schalter 48, 49 an Masse liegen. Ein Stromfluss erfolgt dann von den Klemmen, die die Spannung U₁ bzw. U_{λ/4} führen, über die Schalter 52 bzw. 53 und die Vorwiderstände 54 bzw. 55 jeweils zu Masse, ohne dass der Pockelszellen-Kristall 17PC aktiviert wird. Dadurch erfolgt zu diesem Zeitpunkt keine Drehung der Polarisationsebene des Laserstrahls 9 im Resonator 8 durch die Pockelszelle 17, sondern nur wie erwähnt durch das λ/4-Plättchen 19.

Wenn dann zum Zeitpunkt τ₁ + T_{P} (s. Fig. 6e) das EIN-Schaltsignal S_{ON} wieder auf "0" geht, s. Fig. 6e, wird der Schalter 48 wieder geöffnet, während der Schalter 49 für die restliche Zeit, in der das AUS-Schaltsignal S_{OFF} auf "1" ist, noch geschlossen bleibt. Während dieser Zeitdauer liegt daher ein Stromfluss von der die Spannung U₁ führenden Klemme über den Schalter 52, den Widerstand 54 und den Pockelszellen-Kristall 17PC sowie den geschlossenen Schalter 49 zu Masse vor, d.h. der Laserstrahl 9 wird in seiner Polarisationsebene entsprechend der angelegten Pockelszellen-Spannung U_{PC} = U₁ gedreht, und es werden wiederum Verluste im Laserresonator 8 eingeführt. Wenn das AUS-Schaltsignal S_{OFF} dann ebenfalls auf "0" geht (Zeitpunkt τ₂ + T_{P} gemäß der Darstellung in Fig. 6f), wird auch der Schalter 49 in Fig. 5 geöffnet, und die Pockelszelle 17 liegt dann über die Schalter 52, 53 und die Widerstände 54, 55 an der Differenzspannung U_{λ/4} - U₁. Dementsprechend wird auch während dieser Zeitdauer - bis zum nächsten Einschaltsignal S_{ON} = "1" - an die Pockelszelle 17 eine Spannung (U_{λ/4} - U₁) angelegt, die eine entsprechende Drehung der Polarisationsebene des Laserstrahls 9 und damit Auskopplung des Laserstrahls 9 über den Strahlenteiler 18 bewirkt. Dieser Vorgang ist in Fig. 4b nur während einer Verstärkungsperiode schematisch veranschaulicht, während die übrigen Perioden vereinfacht dargestellt sind.

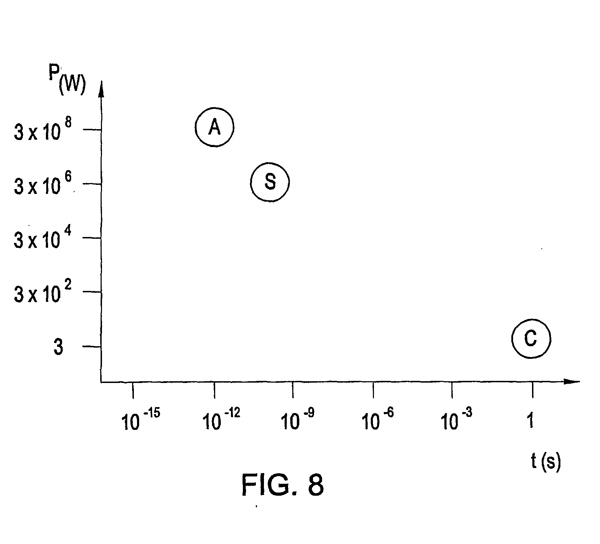

Wie erwähnt wird während der in Fig. 4b dargestellten Zeiten, wo U_{λ/4} an der Pockelszelle 17 anliegt (Zeitintervall τ₂ - τ₁), eine Auskopplung des Laserstrahls 9 verhindert, und während dieser Zeit wird wie erwähnt der Laserpuls im Resonator 8 in 20 bis 30 Durchläufen beträchtlich verstärkt; dies ist auch aus Fig. 7 ersichtlich, in der diese Zunahme der Pulsenergie in der linken Hälfte des Diagramms gezeigt ist, wobei dort die Intensität Iᵣₑₗ über der Zeit aufgetragen ist. Die Dauer τ₂ - τ₁ ist durch die Zeit vorgegeben, bei der die Sättigung der Verstärkung auftritt.

Wenn dann die Spannung an der Pockelszelle 17 auf "0" geht, wird der Laserimpuls zu praktisch 100% aus dem Resonator 8 ausgekoppelt und dem Handstück 2 zugeführt.

Das Spektrum des ausgekoppelten, auf eine hohe Spitzenleistung verstärkten Laserimpulses wird durch Selbstphasenmodulation in der Lichtleiter-Leitung 22 verbreitert, um die Pulsdauer zu verkürzen. Die zwei Gitter 23, 24 (oder Spiegel, Prismen, s. Fig. 2) komprimieren den Impuls auf die gewünschte Dauer (< 1 ps im Fall der Betriebsart A).

Im Handstück 2 kann auf nicht näher dargestellte, keinen Teil der vorliegenden Erfindung bildende Weise eine rasche Bewegung des Laser-Fokus zwecks Vermeidung einer Überhitzung an der Behandlungsstelle 4 vorgesehen werden. In der Betriebsart S ("Oberflächenversiegelung") wird an den Modenkoppler 16 ein HF-Signal mit einer kleineren Leistung P₁ als in der Betriebsart A ("Abtragen") angelegt, in der die Leistung P₂ > P₁ vorgesehen wird. Auf diese Weise wird in der Betriebsart S eine größere Impulsdauer der Laserpulse als in der Betriebsart A erhalten. Durch entsprechende Einstellung der Leistung P_{HF} = P₁ des HF-Signals kann in der Betriebsart S eine Impulsdauer von ca. 100 ps erreicht werden, wogegen in der Betriebsart A wie erwähnt die Impulsdauer in der Größenordnung von 1 ps und darunter beträgt.

In Fig. 8 ist schematisch in einem Diagramm die Spitzenleistung P(W) über der Einwirkzeit t(s) in den drei Betriebsarten A, S und C schematisch veranschaulicht. Dabei ist ersichtlich, dass die Spitzenleistung in der Betriebsart A "Abtragen" am höchsten ist, etwa in der Größenordnung von 3.10⁸ W. Bei einer Impulsdauer < 1 ps bedeutet dies die Aufbringung einer Impulsenergie von ca. 300 µJ. In der Betriebsart S "Oberflächenversiegelung" wird eine Impulsenergie von 300 µJ bei einer Impulsdauer von ungefähr 100 ps - entsprechend einer Spitzenleistung von 3.10⁶ W - aufgebracht.

In der Niederleistungs-Betriebsart C "Koagulieren" wird eine CW-Leistung von ungefähr 3 W während einiger weniger Sekunden aufgebracht.

Zwischen den drei Betriebsarten C, A und S kann mit Hilfe des Fußschalters 7 in komfortabler Weise umgeschaltet werden.

In vielen Anwendungen bei der Zahnbehandlung, ist das Vorsehen eines sichtbaren Pilot-Laserstrahls von Vorteil, da dann der ausgekoppelte Behandlungs-Laserstrahl 3 einfacher auf die richtige Behandlungsstelle 4 gerichtet werden kann. Zur Erzeugung des Pilot-Lasers kann vom therapeutischen Laser 3 durch Frequenzverdopplung ein sichtbarer Laserstrahl hergeleitet werden, oder aber es kann eine zusätzliche Laserquelle, z.B. eine herkömmliche Laserdiode, eingesetzt werden, deren sichtbarer Laserstrahl an einem Spiegel (s. das optische Element 25 in Fig. 2) eingekoppelt wird.

Mehr im Einzelnen wird der sichtbare Pilot-Laserstrahl bevorzugt aus dem Behandlungs-Laserstrahl 3 durch Frequenzverdopplung hergeleitet, wie dies in den Fig. 9 und 10 gezeigt ist. Die Laservorrichtung 1 (s. Fig. 2, 3) arbeitet bei einem fixen Arbeitspunkt und liefert ständig die optische Leistung P_{IR}. Ein kleiner Teil der im infraroten (z.B. bei 1064 nm) liegenden Ausgangsleistung wird nun mittels eines nichtlinearen Kristalls 60 frequenzverdoppelt und somit in den grünen Spektralbereich verschoben, vgl. den Hinweis auf "P_{GR}" in den Fig. 9 und 10. Da nur etwa 1% der Strahlung konvertiert werden muss, um einen gut sichtbaren Pilotstrahl zu generieren, bleibt die Infrarot-(IR)-Ausgangsleistung P_{IR} praktisch unverändert.

Um ferner die IR-Leistung abschwächen zu können und dabei den Pilotstrahl nicht zu verändern, gibt es verschiedene Möglichkeiten. Gemäß Fig. 9 wird eine Scheibe 61 verwendet, die aus einzelnen Teilen 61.1, 61.2....61.n zusammengesetzt ist, und die senkrecht zum Strahlengang verschoben wird, vgl. Pfeil 62 in Fig. 9. Die einzelnen Teile 61.1...61.n bestehen jeweils aus einem Substrat, das mit dielektrischen Multischichten beschichtet ist, deren Eigenschaften auf an sich bekannter Weise durch geeignete Wahl von Schichtdicken und Material über einen weiten Bereich frei eingestellt werden können. Alle Schichten haben gemeinsam, dass sie für die grüne Wellenlänge (z.B. 532 nm) hochtransparent sind; die Reflektivität R für den IR-Strahl wird jedoch von Schicht zu Schicht größer. Die Anzahl der Schichten bestimmt, wie fein die Leistung P_{IR} variiert werden kann. Beispielsweise können mit sechs Schichten folgende Abschwächungen (Faktor k) vorgesehen werden:
Schicht 61.1: R=0 (k=1)
Schicht 61.2: R=20% (k=0,8)
Schicht 61.3: R=40% (k=0,6)
Schicht 61.4: R=60% (k=0,4)
Schicht 61.5: R=80% (k=0,2)
Schicht 61.6: R=100% (k=0)

Gemäß Fig. 10 wird ein Keil 63, der aus einem dotierten Filtermaterial besteht, senkrecht zum Strahl 3 verschoben, s. Pfeil 62. Das Filtermaterial wird so gewählt, dass der grüne Pilotstrahl kaum beeinflusst wird, der IR-Strahl jedoch je nach Dicke des Keils 63 entsprechend abgeschwächt wird (Abschwächungsfaktor k). An der Spitze des Keils 63 wird somit nahezu die gesamte IR-Leistung transmittiert (k=1), an der Basis nahezu komplett absorbiert (k=0). Ein zweiter, undotierter Keil 64 korrigiert die dabei eingeführte Winkeldispersion.

## Patentansprüche

1. Zwischen verschiedenen Betriebsarten umschaltbare Dentallaseranordnung, **gekennzeichnet durch** eine als Laser-Oszillator und als Laser-Verstärker betreibbare Laservorrichtung (1) mit einem Laserresonator (8), der eine Pumpeinheit (12) und einen aktiven Modenkoppler (16) sowie ein selektive Verluste im Resonator (8) einführendes optisches Verlustelement (17') aufweist, und mit einer Laserstrahl-Auskopplungseinheit (21), wobei der Modenkoppler (16) an eine Steuerschaltung (27) angeschaltet ist zum Umschalten zwischen einer zum Koagulieren von weichem Gewebe vorgesehenen CW-Betriebsart (C), in der der Modenkoppler (16) abgeschaltet ist, und zwei Kurzpuls-Betriebsarten (A, S), in denen von der Steuerschaltung (27) HF-Signale mit gleicher Frequenz, jedoch unterschiedlicher Leistung (P₁, P₂) an den Modenkoppler (16) gelegt sind, um Laserimpulse vorbestimmter Dauer zu erzeugen, wobei eine der zwei Kurzpuls-Betriebsarten (A) zum Abtragen von hartem Zahngewebe vorgesehen ist, wogegen die andere Kurzpuls-Betriebsart (S), in der die Laserstrahlung im Vergleich zur erstgenannten Kurzpuls-Betriebsart eine geringere Spitzenleistung aufweist, zum Oberflächenversiegeln von hartem Zahngewebe vorgesehen ist, und wobei ein Ausgang der Steuerschaltung (27) weiters an das ansteuerbare optische Verlustelement (17') zur Steuerung der Verluste im Resonator (8) in den Kurzpuls-Betriebsarten gelegt ist.

2. Dentallaseranordnung nach Anspruch 1, **dadurch gekennzeichnet, dass** das ansteuerbare optische Verlustelement durch einen den Grad der Auskopplung des Laserstrahls (9) steuernden optischen Schalter gebildet ist.

3. Dentallaseranordnung nach Anspruch 1 oder 2, **dadurch gekennzeichnet, dass** das ansteuerbare optische Verlustelement (17') durch eine Pockelszelle (17) in Verbindung mit einem λ/4-Plättchen (19) gebildet ist.

4. Dentallaseranordnung nach Anspruch 3, **dadurch gekennzeichnet, dass** die Steuerschaltung (27) zur Abgabe unterschiedlich hoher Spannungen an die Pockelszelle, je nach eingeschalteter Betriebsart, schaltbar ist.

5. Dentallaseranordnung nach Anspruch 4, **dadurch gekennzeichnet, dass** die Steuerschaltung (27) zur Abgabe gesonderter Spannungs-EIN- und AUS-Schaltsignale (S_{ON}, S_{OFF}) an die Pockelszelle (17) eingerichtet ist.

6. Dentallaseranordnung nach Anspruch 5, **dadurch gekennzeichnet, dass** die EIN- und AUS-Schaltsignale (S_{ON}, S_{OFF}) in der Steuerschaltung (27) aus dem ihr zugeführten HF-Signal (S₁) durch Frequenzteilung und mit Hilfe unterschiedlicher Verzögerungsglieder (44, 45) hergeleitet werden.

7. Dentallaseranordnung nach einem der Ansprüche 1 bis 6, **dadurch gekennzeichnet, dass** die Steuerschaltung (27) einen HF-Generator (28) mit einem regelbaren Leistungsverstärker (33) enthält.

8. Dentallaseranordnung nach einem der Ansprüche 1 bis 7, **dadurch gekennzeichnet, dass** die Steuerschaltung (26) an einen Betriebsarten-Wählschalter, z.B. einen Fußschalter (7), angeschlossen ist.

9. Dentallaseranordnung nach einem der Ansprüche 1 bis 8, **dadurch gekennzeichnet, dass** im Weg des ausgekoppelten Laserstrahls (3) ein die Frequenz eines Teils des Laserstrahls (3) verdoppelnder Frequenzverdoppler (60) zum Vorsehen eines sichtbaren Lichtstrahls vorgesehen ist.

10. Dentallaseranordnung nach einem der Ansprüche 1 bis 9, **dadurch gekennzeichnet, dass** im Weg des ausgekoppelten Laserstrahls (3) ein steuerbarer Abschwächer, z.B. eine Scheibe (61) mit Teilen (61.1 bis 61.n) unterschiedlicher Reflektivität oder ein aus Filtermaterial gebildeter Keil (63), angeordnet ist.

## Claims

1. A dental laser arrangement switchable between different modes of operation, **characterised by** a laser device (1) operable as a laser oscillator and as a laser amplifier, comprising a laser resonator (8), including a pump unit (12) and an active modelocker (16) as well as an optic loss element (17') introducing selective losses in the resonator (8), and further comprising a laser beam-outcoupling unit (21), wherein the modelocker (16) is connected to a control circuit (27) for switching between a CW-mode of operation (C) for coagulating soft tissue, in which the modelocker (16) is switched off, and two short-pulse modes of operation (A, S) in which high frequency signals of equal frequencies, but different powers (P₁, P₂) are applied by the control circuit (27) to the modelocker (16) so as to generate laser pulses of a pre-determined duration, one of the two short-pulse modes of operation (A) being provided for ablation of hard tooth tissue, while the other short-pulse mode of operation (S), in which the laser radiation has a lower peak power in comparison with the first-mentioned short-pulse mode of operation, is provided for surface sealing of hard tooth tissue, and wherein an output of the control circuit (27) furthermore is applied to the activatable optic loss element (17') for controlling the losses in the resonator (8) in the short-pulse modes of operation.

2. A dental laser arrangement according to claim 1, **characterised in that** the activatable optic loss element is formed by an optic switch controlling the degree of out-coupling of the laser beam (9).

3. A dental laser arrangement according to claim 1 or 2, **characterised in that** the activatable optic loss element (17') is formed by a Pockels cell (17) in combination with a λ/4 platelet (19).

4. A dental laser arrangement according to claim 3, **characterised in that** the control circuit (27) is switchable to deliver voltages of different level to the Pockels cell, depending on the mode of operation turned on.

5. A dental laser arrangement according to claim 4, **characterised in that** the control circuit (27) is arranged for delivering separate voltage-ON and -OFF switching signals (S_{ON}, S_{OFF}) to the Pockels cell (17).

6. A dental laser arrangement according to claim 5, **characterised in that** the ON and OFF switching signals (S_{ON}, S_{OFF}) are derived in the control circuit (27) from the high frequency signal (S₁) supplied thereto by dividing the frequency and by means of different delaying elements (44, 45).

7. A dental laser arrangement according to any one of claims 1 to 6, **characterised in that** the control circuit (27) includes a high frequency generator (28) having a controllable power amplifier (33).

8. A dental laser arrangement according to any one of claims 1 to 7, **characterised in that** the control circuit (27) is connected to a mode-of-operation-selection switch, e.g. a foot-operated switch (7).

9. A dental laser arrangement according to any one of claims 1 to 8, **characterised in that** a frequency doubling device (60) providing a visible beam of light for doubling the frequency of a part of the laser beam (3) is provided in the path of the out-coupled laser beam (3).

10. A dental laser arrangement according to any one of claims 1 to 9, **characterised in that** a controllable weakening means, e.g. a disk (61) having parts (61.1 to 61.n) of different reflectivity or a wedge (63) formed of a filter material, is arranged in the path of the out-coupled laser beam (3).

## Revendications

1. Dispositif laser dentaire pouvant être commuté entre différents modes de service, **caractérisé par** un dispositif laser (1) pouvant être exploité comme oscillateur laser et comme amplificateur laser équipé d'un résonateur laser (8), qui présente une unité de pompage (12) et un coupleur de mode (16) actif ainsi qu'un élément absorbant (17') optique introduisant des pertes sélectives dans le résonateur (8), et d'une unité de déclenchement de faisceau laser (21), le coupleur de mode (16) étant connecté à un circuit de commande (27) pour la commutation entre en mode de service en régime continu (C) prévu pour la coagulation de tissu souple, dans lequel le coupleur de mode (16) est déconnecté, et deux modes de service à impulsion brève (A, S), dans lesquels des signaux à haute fréquence avec la même fréquence, mais avec une puissance différente (P₁, P₂) sont envoyés par le circuit de commande (27) au coupleur de mode (16), afin de générer des impulsions laser de durée prédéfinie, l'un des deux modes de service à impulsion brève (A) étant prévu pour l'enlèvement de tissu dentaire dur, alors que l'autre mode de service à impulsion brève (S), dans lequel le rayonnement laser présente une puissance maximale plus faible par rapport au premier mode de service cité à impulsion brève, est prévu pour le scellement superficiel de tissu dentaire dur, et une sortie du circuit de commande (27) étant posée également sur l'élément absorbant (17') optique activable pour la commande des pertes dans le résonateur (8) dans les modes de service à impulsion brève.

2. Dispositif laser dentaire selon la revendication 1, **caractérisé en ce que** l'élément absorbant optique activable est formé par un interrupteur optique contrôlant le degré du déclenchement du faisceau laser (9).

3. Dispositif laser dentaire selon la revendication 1 ou 2, **caractérisé en ce que** l'élément absorbant (17') optique activable est formé par une cellule de Pockels (17) en liaison avec une plaquette quart d'onde (λ/4) (19).

4. Dispositif laser dentaire selon la revendication 3, **caractérisé en ce que** le circuit de commande (27) est commutable, selon le mode de service enclenché, pour l'envoi de hautes tensions, de niveau différent à la cellule de Pockels.

5. Dispositif laser dentaire selon la revendication 4, **caractérisé en ce que** le circuit de commande (27) est aménagé pour l'envoi de signaux de commutation marche et arrêt de tension séparés (S_{ON}, S_{OFF}) à la cellule de Pockels (17).

6. Dispositif laser dentaire selon la revendication 5, **caractérisé en ce que** les signaux de commutation marche et arrêt (S_{ON}, S_{OFF}) sont dérivés dans le circuit de commande (27) du signal à haute fréquence (S₁) qui lui est fourni par division de fréquence et à l'aide de différents éléments de temporisation (44, 45).

7. Dispositif laser dentaire selon l'une quelconque des revendications 1 à 6, **caractérisé en ce que** le circuit de commande (27) contient un oscillateur haute fréquence (28) avec un amplificateur de puissance (33) réglable.

8. Dispositif laser dentaire selon l'une quelconque des revendications 1 à 7, **caractérisé en ce que** le circuit de commande (26) est raccordé à un sélecteur de mode de service, par exemple à un interrupteur à pédale (7).

9. Dispositif laser dentaire selon l'une quelconque des revendications 1 à 8, **caractérisé en ce que**, sur le chemin du faisceau laser (3) déclenché, il est prévu un doubleur de fréquence (60) qui double la fréquence d'une partie du faisceau laser (3) pour la prévision d'un faisceau de lumière visible.

10. Dispositif laser dentaire selon l'une quelconque des revendications 1 à 9, **caractérisé en ce que** sur le chemin du faisceau laser (3) déclenché est disposé un atténuateur contrôlable, par exemple un disque (61) avec des parties (61.1 jusqu'à 61.n) de réflectivité différente ou une clavette (63) formée à base de matériau filtrant.
